(19) Europäisches Patentamt
European Patent Office
Office européen des brevets

(11) EP 4 239 056 A1

(12) **EUROPEAN PATENT APPLICATION**
published in accordance with Art. 153(4) EPC

(43) Date of publication:
**06.09.2023 Bulletin 2023/36**

(21) Application number: **21898551.3**

(22) Date of filing: **23.11.2021**

(51) International Patent Classification (IPC):
***C12N 5/071*** *(2010.01)*

(52) Cooperative Patent Classification (CPC):
**C12N 5/06**

(86) International application number:
**PCT/KR2021/017258**

(87) International publication number:
**WO 2022/114725 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 KR 20200162856**

(71) Applicant: **Xcell Therapeutics Inc.**
**Seoul 08394 (KR)**

(72) Inventors:
- **LEE, Ui Il**
  **Seoul 06326 (KR)**
- **LEE, Joo Youn**
  **Seoul 07324 (KR)**
- **PARK, Ji Soo**
  **Seoul 08602 (KR)**
- **JANG, Ji Eun**
  **Seoul 08728 (KR)**
- **YANG, Yu Yeong**
  **Seoul 08608 (KR)**
- **KANG, Min Hee**
  **Seoul 08354 (KR)**

(74) Representative: **Isarpatent**
**Patent- und Rechtsanwälte Barth**
**Charles Hassa Peckmann & Partner mbB**
**Friedrichstrasse 31**
**80801 München (DE)**

(54) **MEDIUM COMPOSITION FOR DERMAL PAPILLA CELL GROWTH, CONTAINING ROTENONE AND ALBUMIN AS ACTIVE INGREDIENTS**

(57) The present invention relates to a culture medium composition obtained by further adding at least one selected from the group consisting of rotenone and albumin to a serum-free medium prepared through a CAMPs technology. It has been ascertained that the proliferation of dermal papilla cells and maintenance of characteristics thereof during culturing can be improved by regulating the concentration of rotenone and albumin, and since the culture medium composition is a serum-free chemical medium comprising no serum, there is no risk of side effects, which can be caused by using a conventional serum medium, during use on the human body.

The culture medium composition of the present invention obtained by further adding at least one selected from the group consisting of rotenone and albumin to a serum-free medium, or dermal papilla cells cultured through the composition can be effectively used in the development of therapeutic agents for hair loss prevention and hair growth promotion.

EP 4 239 056 A1

【FIG. 4】

CD34

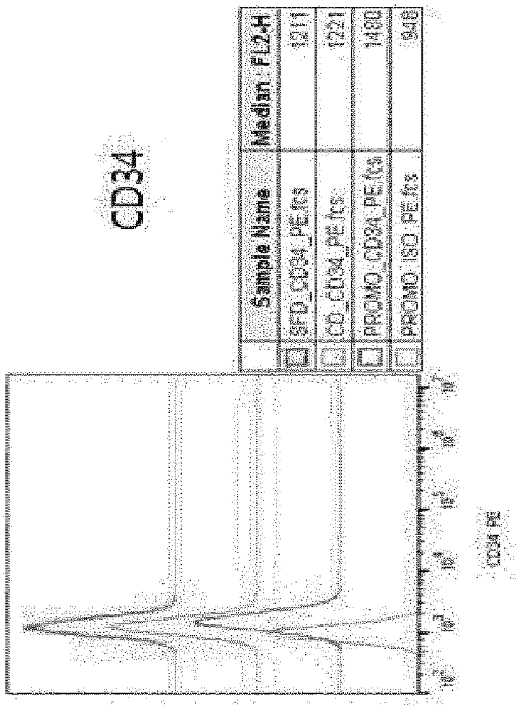


Versican

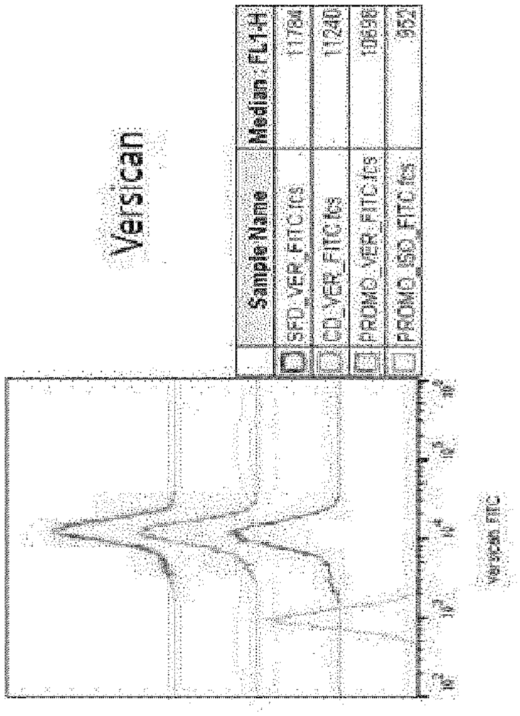


Alkaline Phosphatase

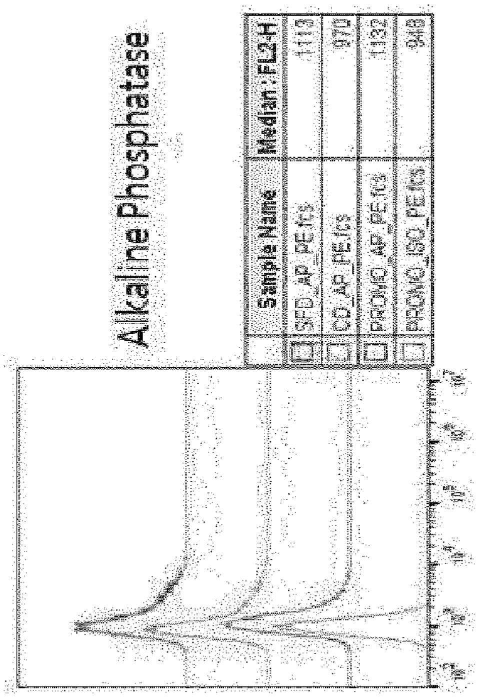


| POSITIVE (%) | CD34 | AP | VERSICAN |
|---|---|---|---|
| SFD | 6.9 | 22.0 | 99.5 |
| CD | 7.3 | 15.7 | 99.7 |
| PROMO | 15.6 | 15.4 | 99.2 |

## Description

### [TECHNICAL FIELD]

The present invention relates to a culture medium composition for growing dermal papilla cells, and more particularly, to a culture medium composition containing rotenone and albumin as active ingredients, which can promote the growth of dermal papilla cells and maintain the characteristics thereof.

### [BACKGROUND ART]

Dermal papilla cells (DPCs) are cells located at the base of the hair follicle, and regulate the growth of hair follicle epithelial cells by secreting growth factors and inhibition factors while being supplied with nutrients through capillaries located at the periphery of the hair follicle. The interaction between epithelial cells in the hair follicle and dermal papilla cells plays an important role in the growth of human hair and this interaction determines the growth cycle of human hair.

Hair goes through a certain hair cycle, but once it passes through the growth stage in which dermal papilla cells actively divide and proliferate, and enters the catagen or telogen stage, the metabolic process slows down while maintaining the shape of the hair, in which dermal papilla cells grow slowly, cell division stops and hair stops growing.

Therefore, in light of the fact that methods of culturing and transplanting new dermal papilla cells, instead of existing dermal papilla cells that grow slowly in this way, can be used for the treatment of hair loss, research on this is being actively conducted, but it is difficult to separate only dermal papilla cells from scalp tissue, and even when cultured in large quantities, there is a limitation in that the ability to induce hair follicle formation and the ability to regenerate hair are reduced in the process of going through passage several times.

In order to solve the above problems, efforts has been made to adjust the components of the culture medium and culture dermal papilla cells, but the culture medium currently mainly used for the culture contains bovine serum and animal-derived components. A cell therapeutic agent produced in a culture medium containing such animal-derived components may cause side effects in the human body, which is problematic. Therefore, there is a need to develop a dermal papilla cell culture medium that does not contain animal serum and thus has a low risk of causing side effects in the human body.

On the other hand, rotenone is a substance that inhibits oxidative phosphorylation in mitochondria in cells, inhibits ATP synthesis, and generates active oxygen. Rotenone is known to inhibit cell division at concentrations of 10-100 nM. Rotenone is considered to be able to be used for culturing dermal papilla cells whose proliferation ability increases in a low-oxygen environment, and thus, some patents confirming their effects in serum media are present (e.g., Korean Patent No. 10-2158584). However, the development of a serum-free medium for dermal papilla cells using rotenone has not yet been confirmed.

### [DETAILED DESCRIPTION OF THE INVENTION]

### [Technical Problem]

The present inventors have made efforts to overcome the difficulties of ex vivo culture of dermal papilla cells and to develop a new serum-free medium for dermal papilla cells with high stability, and as a result, found that the proliferation ability, viability, and hair follicle formation-inducing ability of dermal papilla cells cultured in a serum-free medium containing rotenone are remarkably increased.

Therefore, it is an object of the present invention to provide a culture medium additive for culturing dermal papilla cells, comprising rotenone.

It is another object of the present invention to provide a medium composition for culturing dermal papilla cells, comprising the medium additive and a basal medium.

It is yet another object of the present invention to provide a method of culturing dermal papilla cells, comprising culturing dermal papilla cells in the medium composition.

However, the objects of the present invention are not limited to the aforementioned objects, and other objects which are not mentioned herein will be able to be clearly understood by those skilled in the art from the following detailed description.

### [Technical Solution]

The inventors of the present invention as described above have made efforts to overcome the difficulties of ex vivo culture of dermal papilla cells and to develop a new serum-free medium for dermal papilla cells with high stability,

and as a result, found that the proliferation ability, viability, and hair follicle formation-inducing ability of dermal papilla cells cultured in a serum-free medium containing rotenone are remarkably increased.

Therefore, according to one aspect of the present disclosure, there is provided a culture medium additive for culturing dermal papilla cells, comprising rotenone.

According to another aspect of the present disclosure, there is provided a culture medium composition for culturing dermal papilla cells, comprising the culture medium additive and a basal medium.

According to yet another aspect of the present disclosure, there is provided a method for culturing dermal papilla cells, comprising culturing dermal papilla cells in the culture medium composition.

However, the objects of the present invention are not limited to the aforementioned objects, and other objects which are not mentioned herein will be able to be clearly understood by those skilled in the art from the following detailed description. In order to achieve the above objects, according to the present invention, a culture medium additive for culturing dermal papilla cells, comprising rotenone is provided.

In one embodiment of the present invention, the culture medium additive may further comprise albumin.

In another embodiment of the present invention, the rotenone may be contained at a concentration of 1 to 1000 pM.

In yet another embodiment of the present invention, the albumin may be contained at a concentration of 0.1 g/L to 1 g/L.

In still yet another embodiment of the present invention, the dermal papilla cells may be derived from humans.

Additionally, according to the present invention, a culture medium composition for culturing dermal papilla cells, comprising the culture medium additive and a basal medium is provided.

In one embodiment of the present invention, the culture medium composition may be a serum-free medium composition.

In another embodiment of the present invention, the basal medium may be any one selected from the group consisting of DMEM(Dulbecco's Modified Eagle's Medium); MEM(Minimal Essential Medium); BME(Basal Medium Eagle); RPMI 1640; DMEM/F-10(Dulbecco's Modified Eagle's Medium: Nutrient Mixture F-10); DMEM/F-12(Dulbecco's Modified Eagle's Medium: Nutrient Mixture F-12); a-MEM(a-Minimal essential Medium); G-MEM(Glasgow's Minimal Essential Medium); IMDM(Isocove's Modified Dulbecco's Medium); KnockOut DMEM(GIBCO, USA); and MCDB.

In yet another embodiment of the present invention, the medium composition may maintain the characteristics of dermal papilla cells for at least 10 passages.

Further, according to the present invention, a method for culturing dermal papilla cells, comprising a step of culturing dermal papilla cells in the culture medium composition is provided.

**[Advantageous Effects]**

The present invention relates to a culture medium composition obtained by further adding at least one selected from the group consisting of rotenone and albumin to a serum-free medium prepared through a CAMPs technology. When dermal papilla cells are cultured using this, cell proliferation is promoted, and even if the culture is continued, the characteristics of dermal papilla cells can be maintained, hair loss can be prevented, and hair growth can be promoted.

However, the effects of the present disclosure are not limited to the effects mentioned above, and should be understood to include all effects that can be inferred from the configuration of the invention described in the detailed description or the appended claims.

**[BRIEF DESCRIPTION OF THE DRAWINGS]**

FIG. 1 shows the results of confirming the degree of cell proliferation after treating rotenone at different concentrations in a serum-free medium and then culturing dermal papilla cells.

FIG. 2 confirms the proliferation ability and viability of dermal papilla cells cultured while treating with different concentrations (0.1, 0.5, and 1 g/L) of albumin in a serum-free medium. FIG. 2a is a photograph of dermal papilla cells cultured in a serum-free medium treated with different concentrations of albumin, FIG. 2b is a diagram showing the results of confirming the doubling time of dermal papilla cells passaged in a serum-free medium treated with different concentrations of albumin, FIG. 2c is the result of confirming the cumulative cell number of dermal papilla cells passaged in a serum-free medium treated with different concentrations of albumin, and FIG. 2d is a result of confirming the viability of dermal papilla cells passaged in a serum-free medium treated with different concentrations of albumin.

FIG. 3 confirms the proliferation ability and viability of dermal papilla cells cultured in a serum-free medium treated with 0.1 g/L of albumin in the presence or absence of rotenone (10 pM). FIG. 3a is a photograph of dermal papilla

cells cultured in a serum-free medium in the presence or absence of rotenone, FIG. 3b shows the results of confirming the doubling time of dermal papilla cells passaged in serum-free medium in the presence or absence of rotenone, FIG. 3c is the result of confirming the accumulated cell number of dermal papilla cells passaged in serum-free medium in the presence or absence of rotenone, and FIG. 3d is the result of confirming the viability of dermal papilla cells passaged in serum-free medium in the presence or absence of rotenone.

FIG. 4 confirms whether or not the cultured dermal papilla cells maintain the characteristics of dermal papilla cells depending on the culture conditions in the serum-free medium treated with 0.1 g/L of albumin in the presence or absence of rotenone (10 pM), using biological markers.

## [DETAILED DESCRIPTION OF THE EMBODIMENTS]

The above objects of the present invention are achieved by a culture medium additive for culturing dermal papilla cells, comprising rotenone.

The present inventors have made efforts to overcome the difficulties of ex vivo culture of dermal papilla cells and to develop a new serum-free medium for dermal papilla cells with high stability, and as a result, found that the proliferation ability, viability, and hair follicle formation-inducing ability of dermal papilla cells cultured in a serum-free medium containing rotenone are remarkably increased. The present invention has been completed on the basis of this finding.

Therefore, the present invention provides a culture medium additive for culturing dermal papilla cells, comprising rotenone.

In the present invention, the culture medium additive may further comprise albumin.

In the present invention, the rotenone has a chemical structure represented by the following Chemical Formula 1, and the IUPAC name is (2R,6aS,12aS)-1,2,6,6a,12,12a-hexahydro-2-isopropenyl-8,9-dimethoxychromeno [3,4-b] furo(2,3-h)chromen-6-one. The rotenone is a chemical component obtained from the root of Derris elliptic a, which is a leguminous plant, and is known as a kind of respiratory inhibitor that acts on the respiratory chain of mitochondria. The concentration of rotenone added to the culture medium of the present invention may be 1 to 1000 pM, preferably 10 to 100 pM.

[Chemical Formula 1]

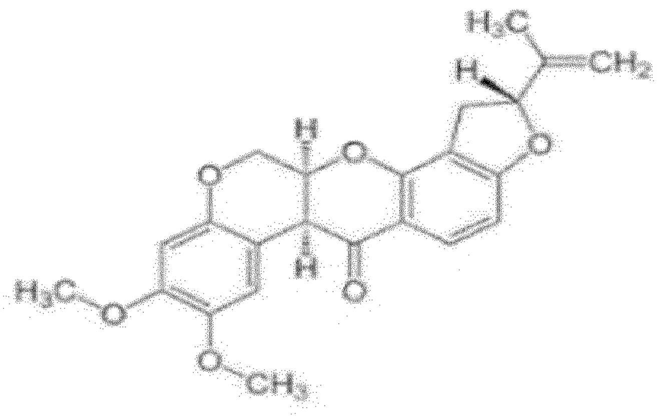

In the present invention, albumin is a type of protein, especially known as a plasma protein that transports various substances including metal ions, drugs and xenobiotic substance. Albumin is also known to be associated with pH regulation and osmotic maintenance of the culture environment. In the present invention, the albumin is not limited thereto, but it can be obtained by extracting from human plasma, serum or the like, or can be produced by recombination. The albumin added to the culture medium of the present invention may have a concentration of 0.1 to 1 g/L, preferably 0.1 to 0.5 g/L.

In one aspect of the present invention, the present invention provides a culture medium composition for culturing dermal papilla cells, comprising the culture medium additive and a basal medium.

In the present invention, the culture medium composition may be a serum-free medium composition, and the serum-free medium is a chemical composition medium to which fetal bovine serum is not added, and may include a basal medium, a growth factor and a core factor as a chemical composition medium to which fetal bovine serum is not added.

In the present invention, the term 'basic medium' is an element that provides the most basic framework of the cell microenvironment so that cells can survive and grow *in vitro,* and may include, but are not limited to, DMEM(Dulbecco's Modified Eagle's Medium); MEM(Minimal Essential Medium); BME(Basal Medium Eagle); RPMI 1640; DMEM/F-10(Dulbecco's Modified Eagle's Medium: Nutrient Mixture F-10); DMEM/F-12(Dulbecco's Modified Eagle's Medium: Nutrient Mixture F-12); a-MEM(a-Minimal essential Medium); G-MEM(Glasgow's Minimal Essential Medium); IMDM(Isocove's Modified Dulbecco's Medium); KnockOut DMEM(GIBCO, USA); or MCDB.

In the present invention, the term 'growth factor' is a protein that binds to a receptor on the surface of a cell and causes proliferation or differentiation of the affected cell, and may include, but are not limited to, cytokines, chemokines, growth factors, neurotrophins or angiogenic factors.

In the present invention, the term 'core factor' is a factor that can regulate cell differentiation, growth, activity, etc., and may include, but are not limited to, amino acids, glucose, salts, vitamins, mixtures of other nutrients, activators, hormones, lipids and related complexes, carriers, vitamins, reducing agents, polyamines, antibodies, or protective additives.

In the present invention, the serum-free medium can be produced using CAMPs technology, which is a technology for producing a culture medium customized for specific cells, the technology comprising the steps of: a) selecting a basal medium; b) selecting growth factors; c) selecting core factors; d) optimizing the concentration and combination ratio; e) scaling up; and f) evaluating equivalence.

In the present invention, the dermal papilla cells may be isolated from humans, mice, hamsters, rats, guinea pigs, monkeys, dogs, cats, rabbits, cows, sheep or pigs, and preferably, it may be derived from humans.

In the present invention, it was confirmed through specific examples whether the culture medium composition according to the present invention can promote the growth of dermal papilla cells and maintain the characteristics of dermal papilla cells.

In one embodiment of the present invention, it was observed that the growth of cultured dermal papilla cells is promoted when treated with rotenone (see Example 1), and the growth of dermal papilla cells was also confirmed when treated with albumin (see Example 2), thus showing that a culture medium capable of promoting the culture of dermal papilla cells can be produced by treating with albumin and rotenone.

In another embodiment of the present invention, the growth of dermal papilla cells was observed in a medium treated with both rotenone and albumin (see Example 3). Based on these results, optimal concentrations of rotenone and albumin suitable for culturing dermal papilla cells were confirmed.

In another embodiment of the present invention, when treated with the optimal concentrations of rotenone and albumin, it was confirmed by flow cytometry that the characteristics of the dermal papilla cells do not disappear even if the culture of the dermal papilla cells is continued, as compared to a control group (see Example 4).

The present inventors have found through the results of the above examples that, when dermal papilla cells are cultured using the culture medium of the present invention, the cell proliferation rate is promoted, the mortality rate is low, and the characteristics of the dermal papilla cells themselves do not disappear even if the culture is continued, and also that the culture medium composition of the present invention and the dermal papilla cells cultured through it can be usefully used in areas such as the treatment and improvement of hair loss.

In yet another aspect of the present invention, the present invention provides a method for culturing dermal papilla cells, comprising culturing dermal papilla cells in the culture medium composition.

Hereinafter, preferred embodiments will be presented in order to help understanding the present invention. However, the following examples are just provided to easily understand the present invention, and the contents of the present invention are not limited thereby.

## [EXAMPLE]

### Example 1. Confirmation of dermal papilla cell proliferation promoting effect due to rotenone treatment

Human dermal papilla cells (hDPC) purchased from PromoCell were inoculated in a Follicle Dermal Papilla cell Growth media (Promocell, Heidelberg, Germany) supplemented with 1% Antibiotic and Antimycotic Hyclone sv30079.91 (hereinafter referred to as Promocell medium), and a serum-free medium, and cultured in a 5% carbon dioxide incubator at 37°C to prepare cells. The prepared human dermal papilla cells (hDPCs) were seeded at a concentration of $2\times10^3$/well into 96-well plates, in which a serum-free medium treated with different concentrations (0, 1, 10, 100, 1000 pM) of rotenone (Tocris Bioscience, UK) was put, in a Follicle Dermal Papilla cell Growth media as a commercially available medium, and then cultured for 72 hours. Then, the culture medium in each well was removed, then added to 10 ul CCK-8 reagent and 90 ul medium, respectively, and cultured for 2 hours at a temperature of 37°C. The absorbance of the supernatant was measured at 450 nm using an ELISA reader to confirm the extent of cell proliferation.

As a result, as shown in FIG. 1, it was confirmed that when treated with rotenone (Tocris Bioscience), the proliferation of dermal papilla cells increases compared to a control group (untreated group) and PromoCell medium in

the concentration range of 10 to 100 pM. Thus, the present inventors have confirmed that a culture medium that promotes the proliferation of dermal papilla cells can be produced by adjusting the concentration of rotenone.

**Example 2. Confirmation of dermal papilla cell proliferation promoting effect according to the albumin concentration**

2-1. Confirmation of proliferation degree of dermal papilla cells according to the albumin concentration

hDPCs were prepared in the same manner as in Example 1. They were treated with different concentrations (0.1, 0.5, 1 g/L) of albumin (Richcore, India) in a serum-free medium containing 1% Antibiotic and Antimycotic Hyclone sv30079.91, and then put in a 25T flask. The cells were inoculated at a concentration of $1.52\times10^5$/flask. After culturing in a 5% carbon dioxide incubator at 37°C, photographs of the cells were taken before passage to confirm the density of dermal papilla cells according to the concentration of albumin (Richcore). As a result, as shown in FIG. 2a, it was confirmed that a lower concentration of albumin (0.1 g/L) exhibits a higher cell density than a high concentration of albumin (1 g/L).

On the other hand, each time the density of the cells proliferated to 90% or more, the cells were passaged. During the passage, the cells were detached from each flask by the use of TrypLE Express, and then the cells were centrifuged. The supernatant was discarded to leave only the cells in the lower layer. The cells were well resuspended by adding a condition-based medium, and then the cell numbers were measured using NC-250™ (Chemometec, Denmark). Using the measured cell number and culture time, the doubling time and accumulated cell number per day were calculated using the following Equation.

A = previous passage, B = current passage

[Equation 1]

$$\text{Doubling time} = \ln(2) \text{ X } (\Delta\text{time between B and A}) / \ln(\text{count B}/ \text{count A})$$

[Equation 2]

$$\text{Accumulated cell number (ACN)} = \text{ACN of A X cell count of B / seeding number}$$

As a result, as shown in FIG. 2b, it was confirmed that a low albumin concentration (0.1 g/L) condition exhibits a lower doubling time than a high albumin concentration (1 g/L) condition, showing that dermal papilla cells show excellent proliferation ability. It was also confirmed the accumulated cell numbers over time appear to be higher in a low albumin concentration condition than in a high albumin concentration condition.

2-2. Confirmation of viability of dermal papilla cells according to the albumin concentration

Each time the density of the cells proliferated to 90% or more, the cells were passaged. During the passage, the cells were detached from each flask by the use of TrypLE Express, and then the cells were centrifuged. The supernatant was discarded to leave only the cells in the lower layer. The cells were well resuspended by adding a condition-based medium, and then while progressing the subculture to 10 passages, the cell viability was measured using NC-250™ (Chemometec, Denmark) at each passage. As a result, as shown in FIG. 2d, it was confirmed that a low albumin concentration (0.1 g/L) condition exhibits higher cell viability than a high albumin concentration (1 g/L) condition.

**Example 3. Confirmation of dermal papilla cell proliferation ability of albumin-added culture medium according to the** presence **or** absence **of rotenone treatment**

3-1. Confirmation of the degree of proliferation of dermal papilla cells according to the presence or absence of rotenone

hDPCs were prepared in the same manner as in Example 1. In the medium supplemented with albumin at a concentration of 0.1 g/L, which showed the best cell growth ability and viability in the results of Example 2, a serum-free media supplemented with 1% Antibiotic and Antimycotic Hyclone sv30079.91 in the presence or absence of 10 pM

rotenone was prepared, and put in a 25T flask. The hDPCs were inoculated at a concentration of $1.52\times10^5$/flask. After culturing in a 5% carbon dioxide incubator at 37°C, photographs of the cells were taken before passage to confirm the density of dermal papilla cells according to the rotenone treatment. As a result, as shown in FIG. 3a, a higher cell density was observed in the rotenone-treated group than in the rotenone-untreated group.

On the other hand, each time the density of the cells proliferated to 90% or more, the cells were passaged. During the passage, the cells were detached from each flask by the use of TrypLE Express, and then the cells were centrifuged. The supernatant was discarded to leave only the cells in the lower layer. The cells were well resuspended by adding a condition-based medium, and then the cell numbers were measured using NC-250™ (Chemometec, Denmark). Using the measured cell number and culture time, the doubling time and accumulated cell numbers per day were calculated using the following Equation.

A = previous passage, B = current passage

[Equation 1]

$$\text{Doubling time} = \ln(2) \text{ X } (\Delta\text{time between B and A}) / \ln(\text{count B}/\text{ count A})$$

[Equation 2]

$$\text{Accumulated cell number (ACN)} = \text{ACN of A X cell count of B} / \text{seeding number}$$

As a result, it was confirmed that a rotenone (10 pM)-treated group exhibits a lower doubling time than a rotenone-untreated group as shown in FIG. 3b, confirming that the dermal papilla cells show excellent proliferation ability. It was also confirmed the accumulated cell numbers over time appear to be higher in a rotenone-treated group than in a rotenone-untreated group as shown in FIG. 3c.

3-2. Confirmation of viability of dermal papilla cells according to the presence or absence of rotenone treatment.

Each time the density of the cells proliferated to 90% or more, the cells were passaged. During the passage, the cells were detached from each flask by the use of TrypLE Express, and then the cells were centrifuged. The supernatant was discarded to leave only the cells in the lower layer. The cells were well resuspended by adding a condition-based medium, and then the cell viability was measured using NC-250™ (Chemometec, Denmark) at each passage. As a result, as shown in FIG. 3d, it was confirmed that a rotenone-treated group exhibits higher cell viability than a rotenone-untreated group.

**Example 4. Influence of the presence or absence of rotenone on dermal papilla cell characteristics in serum-free chemical composition medium**

hDPCs were prepared in the same manner as in Example 1, and inoculated at a concentration of $1.52\times10^5$/flask in a 75T flask to which a serum-free culture medium treated with 0.1 g/L of albumin and 1 to 100 pM of rotenone was added. The cells were detached from each flask by the use of TrypLE Express, and then the cells were centrifuged. The supernatant was discarded to leave only the cells in the lower layer. The cells were fixed for 15 minutes using a 4% formaldehyde solution, and then reacted on ice for 10 minutes using a 0.5% tween-20 permeabilization solution to prepare cells. The prepared cells were well resuspended in PBS supplemented with 1% FBS, and then the cells were stained using an antibody serving as a previously selected biological marker. CD34 and HLA-DR were selected as negative markers, and alkaline phosphatase, versican, and CD105 were selected as positive markers. After fixing the stained cells with 4% formaldehyde solution for 15 minutes, and flow cytometry was performed using CytoFLEX (Beckman Coulter, USA). As a result, as shown in FIG. 4, it was confirmed that in dermal papilla cells cultured in serum-free culture medium supplemented with rotenone and albumin, biological positive markers exhibiting dermal papilla cell characteristics are maintained at higher levels, and negative markers are maintained at lower levels, as compared to the control group not treated with rotenone.

It is understood that the above-described embodiments are only illustrative of the application of the principles of the present invention. The present invention may be embodied in other specific forms without departing from its spirit or essential characteristics. The described embodiment is to be considered in all respects only as illustrative and not restrictive.

## Claims

1. A culture medium additive for culturing dermal papilla cells, comprising rotenone.

2. The culture medium additive according to claim 1, wherein:
the culture medium additive further comprises albumin.

3. The culture medium additive according to claim 1, wherein:
the rotenone is contained at a concentration of 1 to 1000 pM.

4. The culture medium additive according to claim 2, wherein:
the albumin is contained at a concentration of 0.1 g/L to 1 g/L.

5. The culture medium additive according to claim 1, wherein:
the dermal papilla cells are derived from humans.

6. A culture medium composition for culturing dermal papilla cells, comprising the culture medium additive of claim 1 and a basal medium.

7. The culture medium composition according to claim 6, wherein:
the culture medium composition is a serum-free medium composition.

8. The culture medium composition according to claim 6, wherein:
the basal medium is any one selected from the group consisting of DMEM(Dulbecco's Modified Eagle's Medium); MEM(Minimal Essential Medium); BME(Basal Medium Eagle); RPMI 1640; DMEM/F-10(Dulbecco's Modified Eagle's Medium: Nutrient Mixture F-10); DMEM/F-12(Dulbecco's Modified Eagle's Medium: Nutrient Mixture F-12); a-MEM(a-Minimal essential Medium); G-MEM(Glasgow's Minimal Essential Medium); IMDM(Isocove's Modified Dulbecco's Medium); KnockOut DMEM(GIBCO, USA); and MCDB.

9. The culture medium composition according to claim 6, wherein:
the medium composition maintains the characteristics of dermal papilla cells for at least 10 passages.

10. A method for culturing dermal papilla cells, comprising culturing dermal papilla cells in the culture medium composition of claim 6.

【FIG. 1】

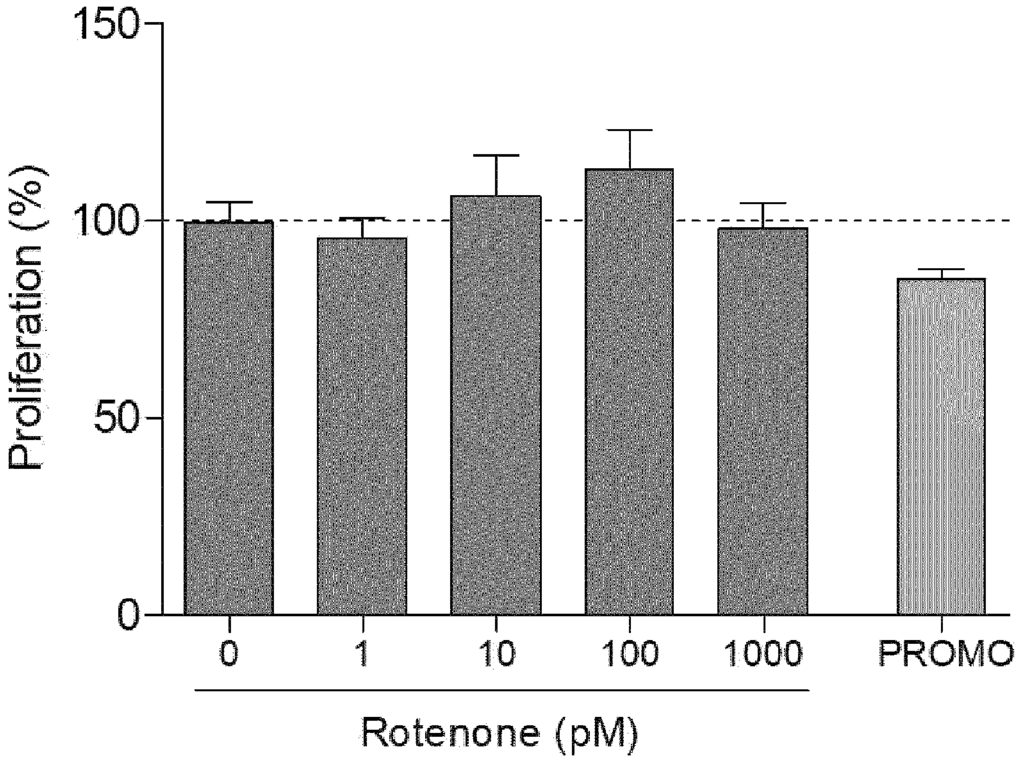

【FIG. 2a】

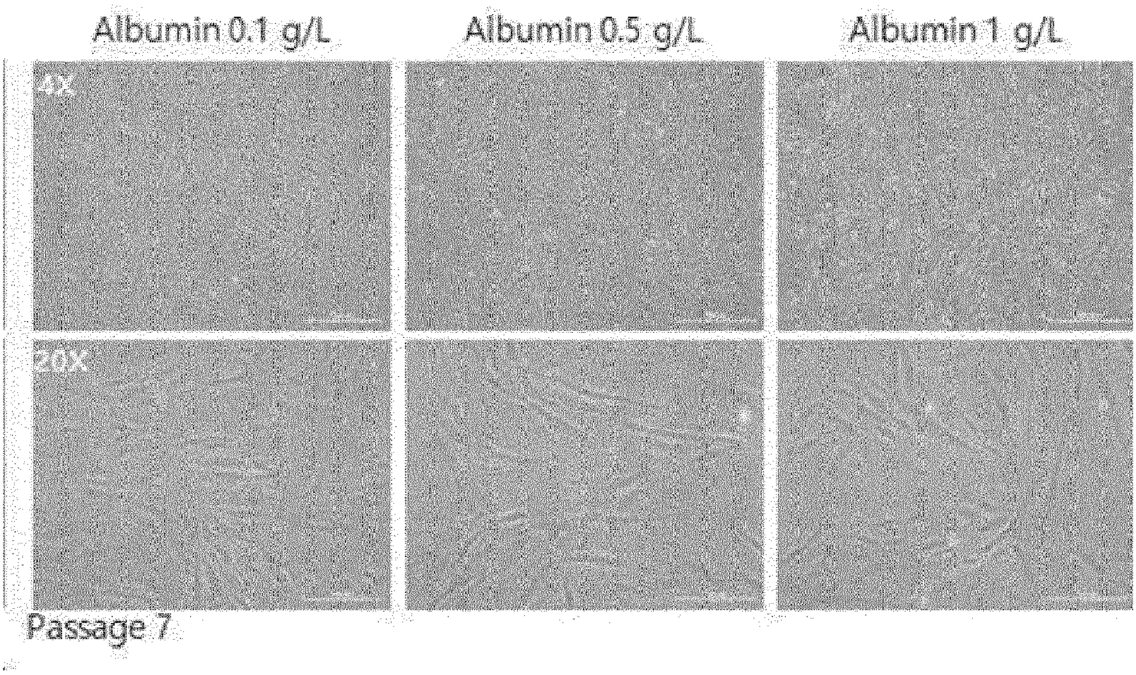
Albumin 0.1 g/L
Albumin 0.5 g/L
Albumin 1 g/L
4X
20X
Passage 7

【FIG. 2b】

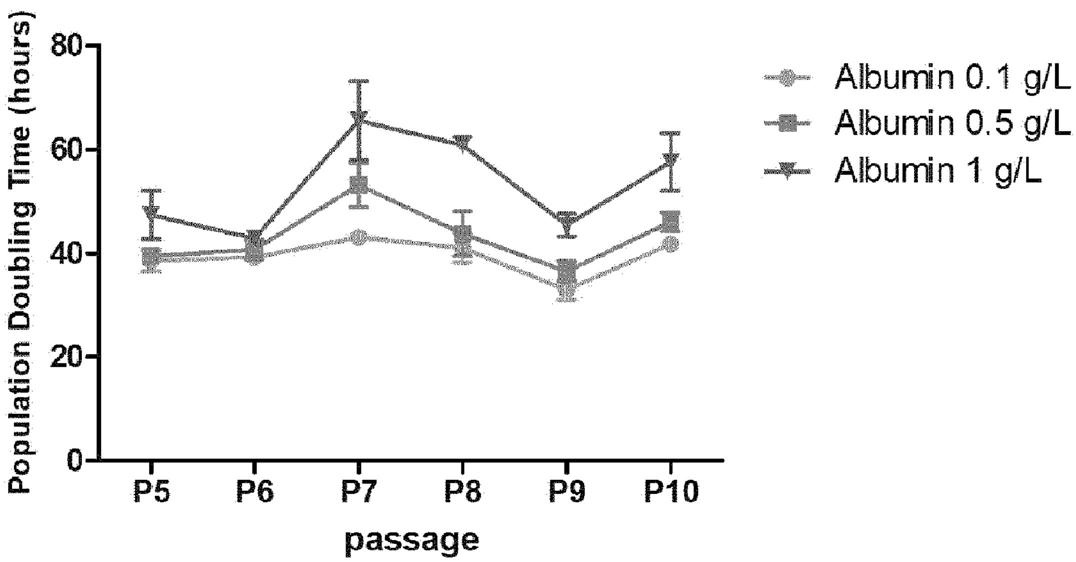
Population Doubling Time (hours)
80
60
40
20
0
P5
P6
P7
P8
P9
P10
passage
Albumin 0.1 g/L
Albumin 0.5 g/L
Albumin 1 g/L

【FIG. 2c】

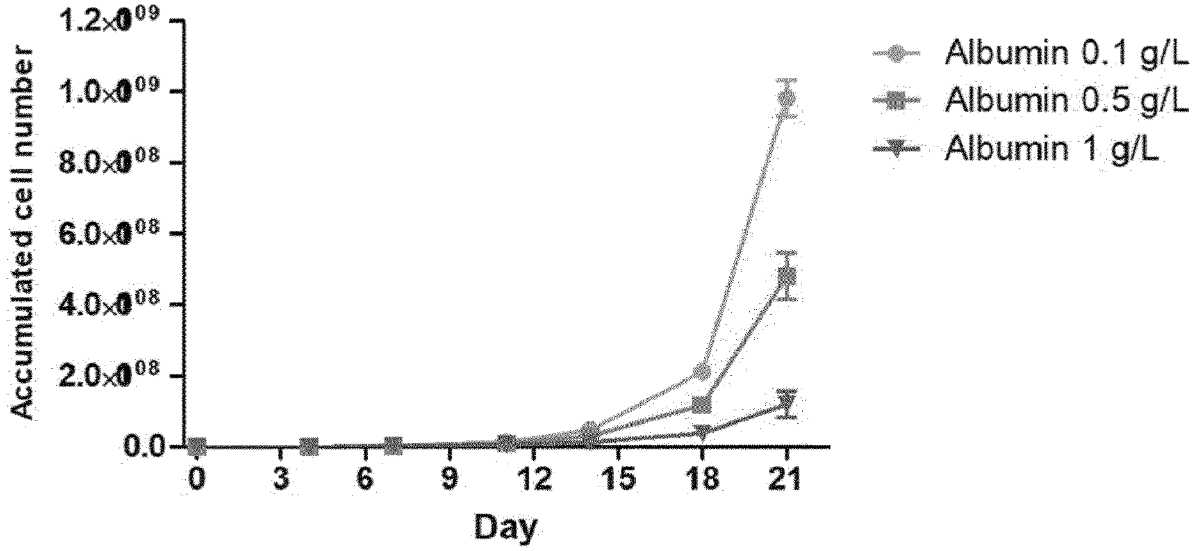
Accumulated cell number
1.2×10^09
1.0×10^09
8.0×10^08
6.0×10^08
4.0×10^08
2.0×10^08
0.0
0
3
6
9
12
15
18
21
Day
Albumin 0.1 g/L
Albumin 0.5 g/L
Albumin 1 g/L

【FIG. 2d】

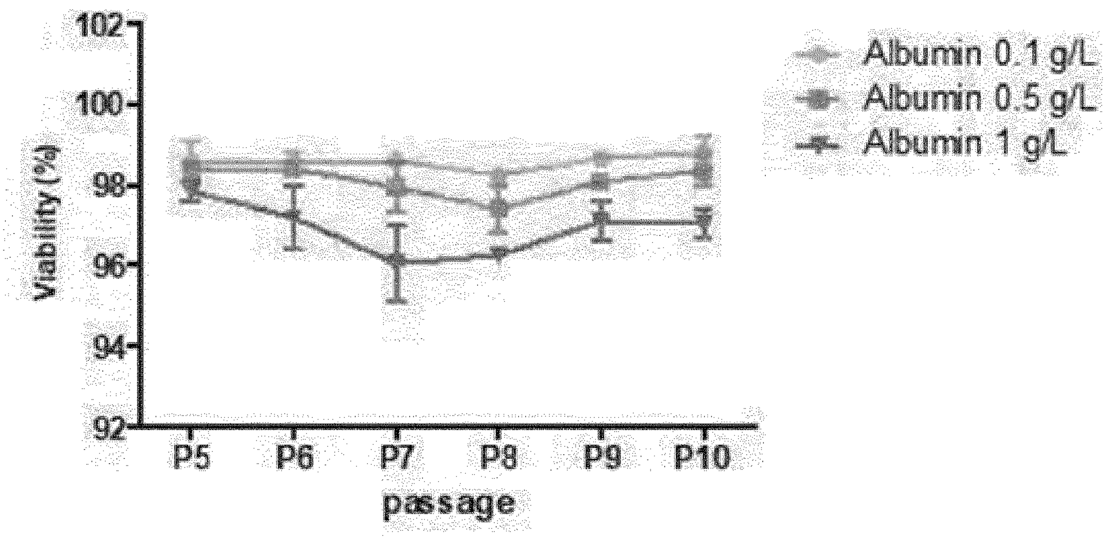
Viability (%)
102
100
98
96
94
92
P5
P6
P7
P8
P9
P10
passage
Albumin 0.1 g/L
Albumin 0.5 g/L
Albumin 1 g/L

【FIG. 3a】

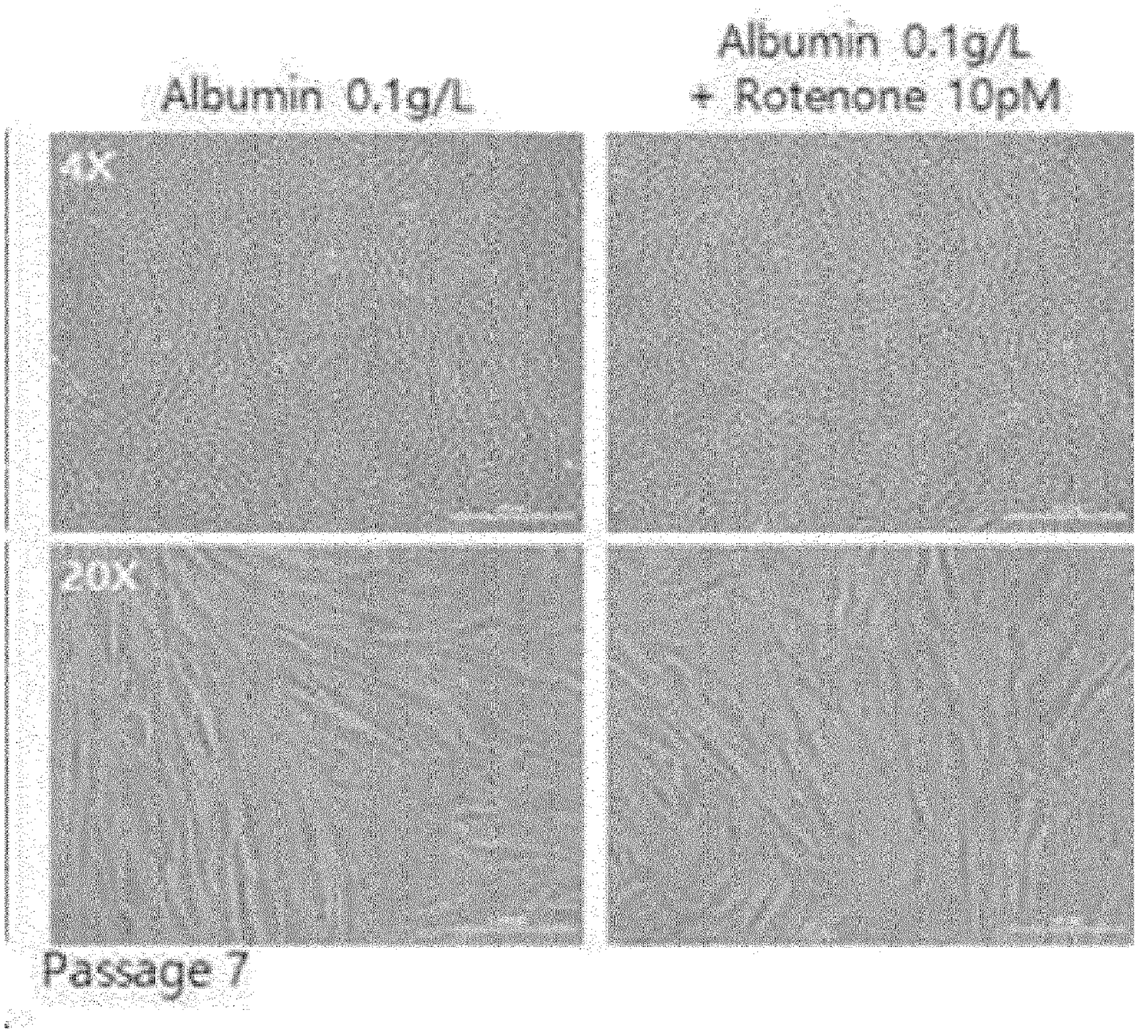

【FIG. 3b】

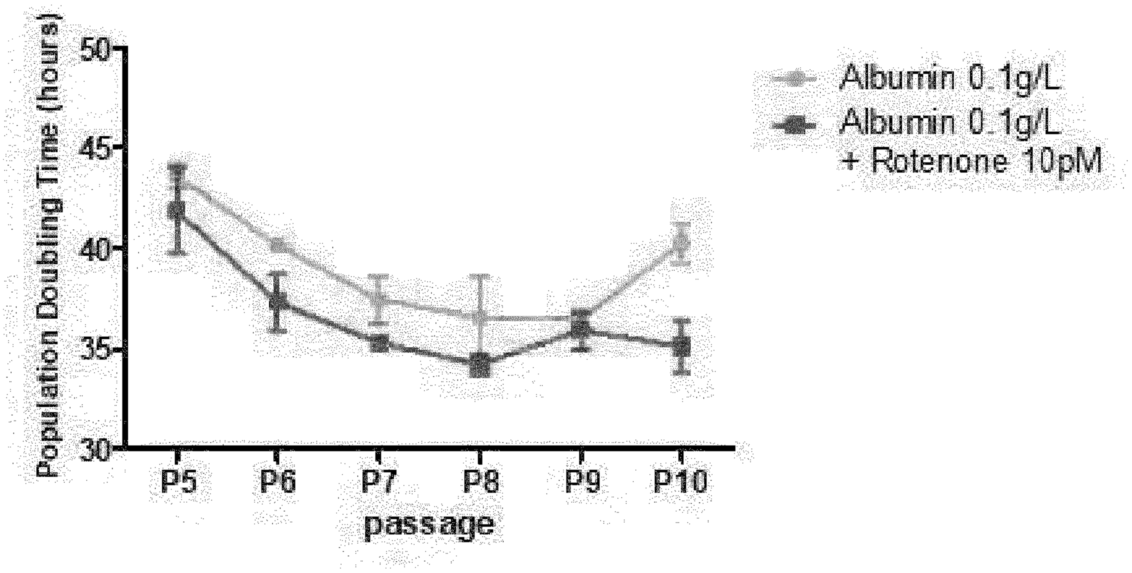
Population Doubling Time (hours)
50
45
40
35
30
P5
P6
P7
P8
P9
P10
passage
Albumin 0.1g/L
Albumin 0.1g/L
+ Rotenone 10pM

【FIG. 3c】

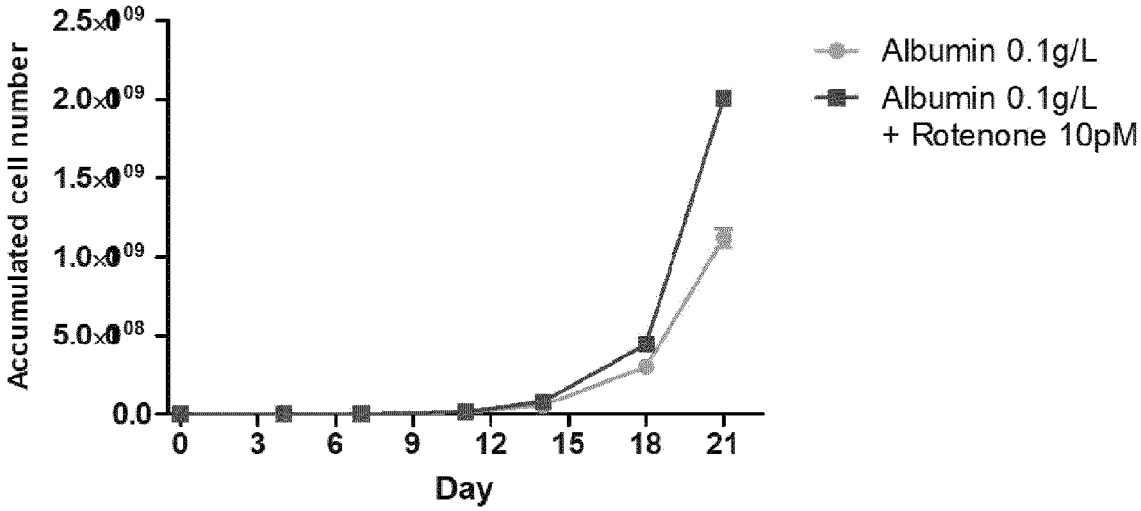
Accumulated cell number
2.5×10^09
2.0×10^09
1.5×10^09
1.0×10^09
5.0×10^08
0.0
0
3
6
9
12
15
18
21
Day
Albumin 0.1g/L
Albumin 0.1g/L
+ Rotenone 10pM

【FIG. 3d】

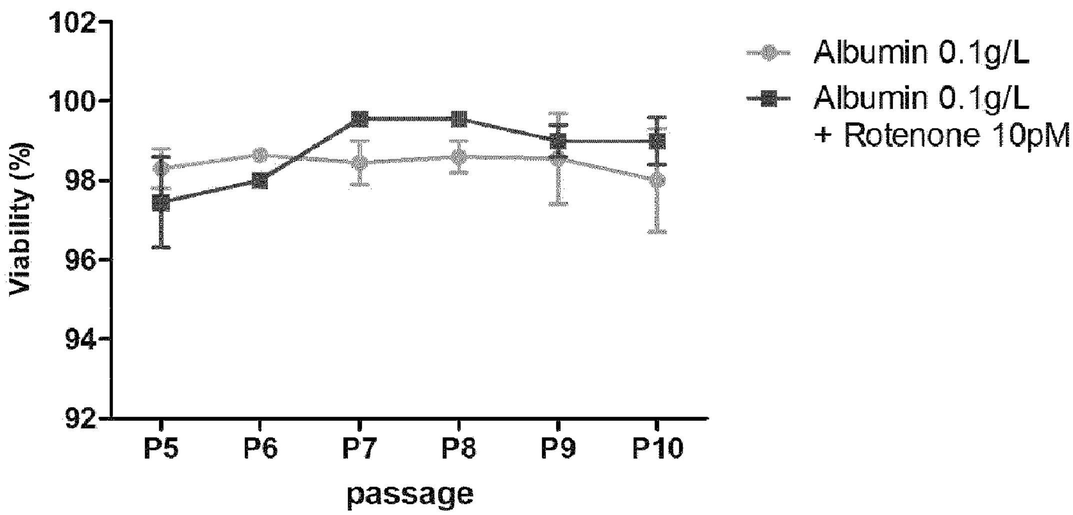
102
100
98
96
94
92
Viability (%)
P5
P6
P7
P8
P9
P10
passage
Albumin 0.1g/L
Albumin 0.1g/L
+ Rotenone 10pM

【FIG. 4】

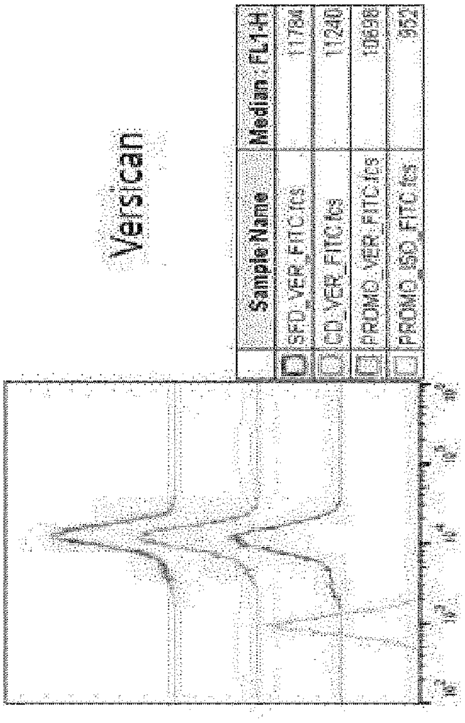


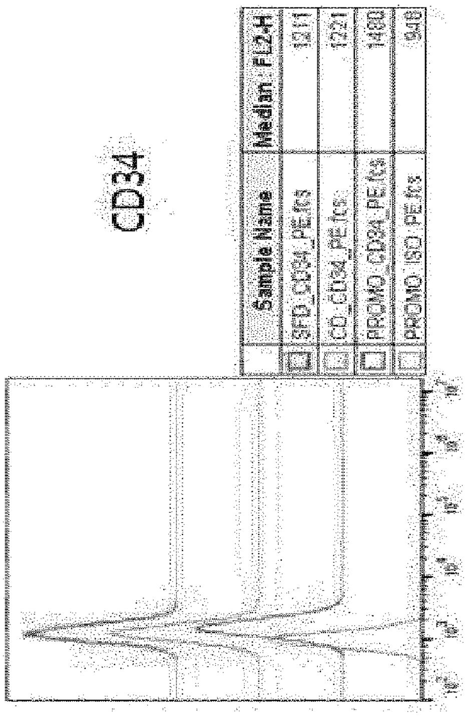


| POSITIVE (%) | CD34 | AP | VERSICAN |
|---|---|---|---|
| SFD | 6.9 | 22.0 | 99.5 |
| CD | 7.3 | 15.7 | 99.7 |
| PROMO | 15.6 | 15.4 | 99.2 |

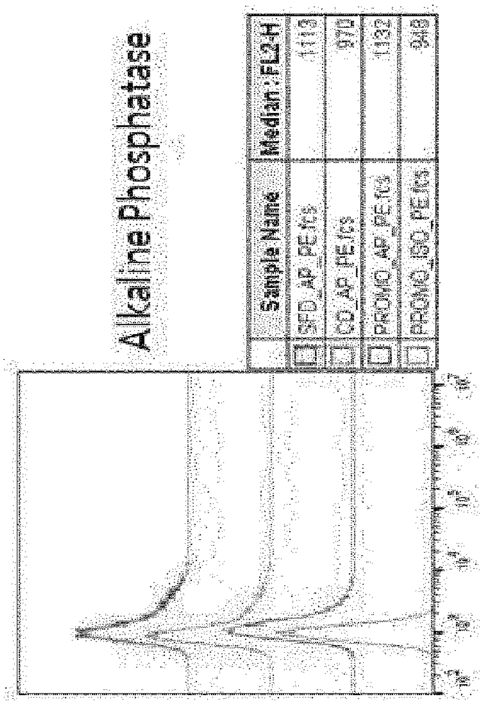

**INTERNATIONAL SEARCH REPORT**

International application No.
**PCT/KR2021/017258**

**A. CLASSIFICATION OF SUBJECT MATTER**

**C12N 5/071**(2010.01)i

According to International Patent Classification (IPC) or to both national classification and IPC

**B. FIELDS SEARCHED**

Minimum documentation searched (classification system followed by classification symbols)

C12N 5/071(2010.01); C12N 5/02(2006.01); C12N 5/07(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 로테논(rotenone), 알부민(albumin), 모유두세포(dermal papilla cell), 배지(medium), 무혈청(serum-free)

**C. DOCUMENTS CONSIDERED TO BE RELEVANT**

| Category* | Citation of document, with indication, where appropriate, of the relevant passages | Relevant to claim No. |
|---|---|---|
| DX | KR 10-2020-0074922 A (STEMORE CO., LTD.) 25 June 2020 (2020-06-25) See paragraph [0081]; and claims 1 and 3. | 1,3,5,6,9,10 |
| DY | | 2,4,7,8 |
| Y | KR 10-2020-0025210 A (STEMORE CO., LTD.) 10 March 2020 (2020-03-10) See abstract; and claims 1 and 5. | 2,4,7,8 |
| X | ZHENG, M. et al. Hypoxia improves hair inductivity of dermal papilla cells via nuclear NADPH oxidase 4-mediated reactive oxygen species generation. British Journal of Dermatology. 2019, vol. 181, pp. 523–534. See abstract; pages 529-530; and figure 5. | 1,3,5,6,9,10 |
| A | KR 10-2006-0059557 A (PARK, Jung Keug) 02 June 2006 (2006-06-02) See entire document. | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 March 2022** | **02 March 2022** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT

International application No.
PCT/KR2021/017258

C. DOCUMENTS CONSIDERED TO BE RELEVANT

| Category* | Citation of document, with indication, where appropriate, of the relevant passages | Relevant to claim No. |
|---|---|---|
| A | KR 10-2010-0004966 A (DONGGUK UNIVERSITY INDUSTRY-ACADEMIC COOPERATION FOUNDATION) 13 January 2010 (2010-01-13) See entire document. | 1-10 |

Form PCT/ISA/210 (second sheet) (July 2019)

**INTERNATIONAL SEARCH REPORT**
**Information on patent family members**

International application No.
**PCT/KR2021/017258**

| Patent document cited in search report | Publication date (day/month/year) | Patent family member(s) | Publication date (day/month/year) |
|---|---|---|---|
| KR 10-2020-0074922 A | 25 June 2020 | KR 10-2019-0103057 A | 04 September 2019 |
| | | KR 10-2020-0109281 A | 22 September 2020 |
| | | KR 10-2138241 B1 | 28 July 2020 |
| | | KR 10-2158584 B1 | 22 September 2020 |
| KR 10-2020-0025210 A | 10 March 2020 | WO 2020-045836 A1 | 05 March 2020 |
| KR 10-2006-0059557 A | 02 June 2006 | AT 408671 T | 15 October 2008 |
| | | CN 101068920 A | 07 November 2007 |
| | | CN 101068920 B | 15 December 2010 |
| | | EP 1819810 A1 | 22 August 2007 |
| | | EP 1819810 B1 | 17 September 2008 |
| | | KR 10-0616752 B1 | 31 August 2006 |
| | | US 2008-0145929 A1 | 19 June 2008 |
| | | US 7635589 B2 | 22 December 2009 |
| | | WO 2006-057542 A1 | 01 June 2006 |
| KR 10-2010-0004966 A | 13 January 2010 | AU 2007-275048 A1 | 24 January 2008 |
| | | AU 2007-275048 B2 | 03 November 2011 |
| | | CA 2656967 A1 | 24 January 2008 |
| | | CA 2656967 C | 02 April 2013 |
| | | CN 101491060 A | 22 July 2009 |
| | | CN 101491060 B | 12 March 2014 |
| | | CN 101755046 A | 23 June 2010 |
| | | CN 101755046 B | 08 August 2012 |
| | | EP 2052520 A2 | 29 April 2009 |
| | | EP 2052520 B1 | 15 August 2018 |
| | | EP 2183358 A1 | 12 May 2010 |
| | | EP 2183358 B1 | 27 March 2013 |
| | | IL 196075 B | 28 November 2013 |
| | | JP 2009-545247 A | 17 December 2009 |
| | | JP 2010-534072 A | 04 November 2010 |
| | | JP 2013-059051 A | 28 March 2013 |
| | | JP 5155312 B2 | 06 March 2013 |
| | | JP 5349474 B2 | 20 November 2013 |
| | | JP 5619846 B2 | 05 November 2014 |
| | | KR 10-1019921 B1 | 08 March 2011 |
| | | KR 10-1022032 B1 | 16 March 2011 |
| | | KR 10-2010-0110905 A | 13 October 2010 |
| | | KR 10-2010-0111325 A | 14 October 2010 |
| | | TW 200816690 A | 01 April 2008 |
| | | TW I368407 B | 11 July 2012 |
| | | US 2008-0020776 A1 | 24 January 2008 |
| | | US 2010-0261276 A1 | 14 October 2010 |
| | | US 8017390 B2 | 13 September 2011 |
| | | US 8391873 B2 | 05 March 2013 |
| | | WO 2008-011610 A2 | 24 January 2008 |
| | | WO 2008-011610 A3 | 20 March 2008 |
| | | WO 2009-014272 A1 | 29 January 2009 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 102158584